# FASCICULE DE BREVET EUROPEEN

(11) **EP 0 862 423 B1**
(45) Date de publication et mention de la délivrance du brevet: **15.09.2004**
(21) Numéro de dépôt: 97928275.3
(22) Date de dépôt: 30.06.1997
(51) Int. Cl.: A61K 9/48

(54) **COMPOSITION PHARMACEUTIQUE POUR ADMINISTRATION PAR VOIE ORALE**
ORAL ANZUWENDENDE ARZNEIZUBEREITUNG
PHARMACEUTICAL COMPOSITION FOR ORAL ADMINISTRATION

(30) Priorité: 04.07.1996 NL 1003503
(43) Date de publication de la demande: 09.09.1998
(73) Titulaire: Negma-Steba International Development N.V., 2514 JB Den Haag (NL)
(72) Inventeur: METZIGER, Pierre, F-67450 Lampertheim (FR); COHEN, Avraham, Tel Aviv (IL)
(74) Mandataire: L'Helgoualch, Jean
(86) Numéro de dépôt international: PCT/EP1997/003405
(87) Numéro de publication internationale: WO 1998/001118

(56) Documents cités:
- EP-A- 0 264 989
- EP-A- 0 364 944
- EP-A- 0 519 428
- EP-A- 0 598 337

## Description

La présente invention concerne une composition pharmaceutique adaptée à une administration par voie orale, et plus particulièrement une composition pharmaceutique adaptée à un principe actif de médicament à usage humain ou vétérinaire, insoluble ou peu soluble dans l'eau et dans les huiles, tel que la diacéréine (diacétylrhéine), la rhéine ou un de leurs sels, permettant d'en améliorer la biodisponibilité.

La diacéréine, décrite dans le brevet FR-A-2.508.798, est un composé dont les propriétés pharmacologiques sont connues depuis plusieurs années, et ont permis son utilisation comme médicament anti-inflammatoire non stéroïdien applicable dans le traitement de maladies telles que l'arthrose. Cependant, la diacéréine est pratiquement insoluble dans l'eau et dans les alcools, ce qui constitue un inconvénient pour son administration, notamment par injection. D'autre part, lorsqu'elle est administrée par voie orale, la diacéréine n'est pas totalement absorbée par le tube digestif, et en raison de cette absorption incomplète, des effets secondaires indésirables peuvent être observés, par exemple des effets laxatifs.

Divers dérivés, compositions pharmaceutiques et formes galéniques destinés à améliorer les propriétés de la diacéréine ou d'autres médicaments insolubles ou peu solubles dans l'eau ont été décrits dans la littérature. Ainsi, par exemple, le brevet EP-A-243.968 décrit un sel de potassium de diacéréine soluble dans l'eau et utilisable dans la préparation de compositions pour administration parentérale.

Le brevet EP-A-598.337 décrit une composition comprenant un principe actif de médicament peu ou pas soluble dans l'eau incorporé dans un polymère réticulé, un agent tensioactif et une huile, présentant une biodisponibilité améliorée. Les brevets US-A-5.225.192 et EP 0364944 décrivent un procédé de préparation d'un médicament à dissolution rapide consistant à incorporer le médicament dans des particules de polymère réticulé insoluble mais susceptible de se gonfler dans l'eau, à le mettre en contact avec un solvant organique, et à sécher sous vide.

La présente invention a pour objet une composition pharmaceutique adaptée à un principe actif de médicament à usage humain ou vétérinaire, contenant un médicament insoluble ou faiblement soluble dans l'eau et les huiles, notamment la diacéréine, la rhéine ou un de leurs sels, administrable par voie orale et procurant une absorption plus rapide et plus complète du principe actif dans l'organisme, et présentant une meilleure biodisponibilité permettant de supprimer ou diminuer les effets secondaires précités.

La présente invention a encore pour objet une nouvelle composition pharmaceutique à base de diacéréine, de rhéine ou de sels de diacéréine ou de rhéine, administrable par voie orale sous forme de capsule ou de gélule, présentant une biodisponibilité améliorée par rapport aux formes usuelles.

La composition pharmaceutique suivant la présente invention, administrable par voie orale, contenant le médicament précité insoluble ou faiblement soluble dans l'eau et les huiles, choisi parmi la diacéréine, la rhéine ou un de leurs sels pharmaceutiquement acceptables, se distingue essentiellement en ce qu'elle comprend
- une huile liquide support, choisie parmi une huile végétale, animale ou minérale,
- un agent de suspension,
- un agent homogénéiseur,
- un surfactif,
en mélange avec le principe actif du médicament et un ou plusieurs excipients ou supports pharmaceutiquement acceptables.

Les sels pharmaceutiquement acceptables utilisables dans l'invention peuvent être par exemple les sels de sodium ou de potassium de diacéréine ou de rhéine.

L'huile liquide support utilisée dans la composition de l'invention peut être choisie parmi une huile végétale (par exemple une huile d'arachide, de soja ou de tournesol), une huile minérale (par exemple une paraffine) et une huile animale. Elle peut être constituée par un ou plusieurs triglycérides à chaîne moyenne. L'expression "chaîne moyenne" employée ici à propos du triglycéride signifie une chaîne linéaire ou ramifiée comprenant de préférence entre 8 et 12 atomes de carbone environ. Bien entendu, il est possible d'utiliser un seul ou plusieurs triglycérides en combinaison. Le triglycéride à chaîne moyenne utilisé dans la composition de l'invention peut être par exemple une huile de coco fractionnée.

L'agent de suspension utilisé dans la composition peut être toute huile solide ou semi-solide à température ambiante, et par exemple une huile végétale hydrogénés, une cire (par exemple la cire d'abeille) ou un gélifiant (par exemple une silice). Suivant l'invention, on utilise de préférence une huile végétale totalement ou partiellement hydrogénée, par exemple l'huile de soja hydrogénée.

Le surfactif peut être un surfactif anionique ou non ionique, mais il est de préférence un surfactif non ionique choisi parmi les esters de sorbitane polyalkylés, des dérivés de polyoxyéthylène sorbitane, et par exemple un ester mono- oléique de sorbitane polyhydroxyéthylé comme le Polysorbate 80 (ou Tween 80®), ou le Span®. Ces surfactifs sont bien connus dans la technique pharmaceutique et disponibles dans le commerce.

L'agent homogénéiseur peut être choisi parmi les agents couramment utilisés dans la technique. Suivant l'invention, on utilise de préférence la lécithine de soja, qui présente l'avantage d'exercer à la fois une action d'homogénéiseur et de fluidifiant pour les composants utilisés.

Les quantités des divers composants de la composition suivant l'invention peuvent être ajustées en fonction des effets recherchés et du principe actif de médicament utilisé.

Dans le cas de la diacéréine et de la rhéine, il peut être particulièrement avantageux d'utiliser une composition comprenant entre 5 et 25% en poids, de préférence entre 5 et 10% en poids, d'agent homogénéiseur, et entre 5 et 25% en poids, de préférence entre 10 et 15% en poids, de surfactif, par rapport au poids total des composants et du principe actif.

Le rapport en poids entre l'huile liquide support et l'agent de suspension est compris entre 8:1 et 2:1, et il est de préférence voisin de 4:1. Ces deux composants peuvent se mélanger à température ambiante ou à chaud en cours de préparation de la composition pour former une pâte ou une pommade. La température est ajustée en fonction de la nature des composants utilisés.

Les composants ci-dessus sont mélangés avec le principe actif de médicament suivant les techniques utilisées habituellement pour les préparations pharmaceutiques, en y ajoutant le cas échéant divers excipients et additifs usuels de mise sous forme pharmaceutique.

Par exemple, dans le cas d'une huile liquide support constituée par des triglycérides à chaîne moyenne et d'un agent de suspension constitué par une huile de soja hydrogénée, associés à de la lécithine de soja comme agent homogénéiseur, on procède de préférence de la manière suivante : on chauffe les triglycérides à une température de 65-70°C environ, on y fait fondre l'huile de soja, et, après refroidissement à température ambiante, on ajoute la lécithine de soja et un surfactif non ionique et le mélange est homogénéisé dans un mélangeur de type usuel, puis on ajoute la diacéréine et on maintient l'homogénéisation du mélange pendant 10 à 30 minutes.

La composition suivant l'invention peut être avantageusement présentée sous forme de capsules molles de gélatine ou sous forme de gélules, contenant par exemple entre 20 et 50 mg de principe actif par unité.

Les capsules et gélules sont préparées suivant les techniques pharmaceutiques usuelles, et peuvent par exemple comporter une paroi à base de gélatine contenant divers additifs tels que de la glycérine, du dioxyde de titane ou de l'oxyde de fer. Il est particulièrement avantageux que la paroi des capsules ou des gélules contienne une substance susceptible d'isoler la composition de toute source d'humidité qui pourrait entraîner une dégradation du principe actif. On peut utiliser une huile silicone, et par exemple de la diméthicone.

Dans le cas de la diacéréine, qui est pratiquement insoluble dans l'eau et dans l'éthanol, et peu soluble dans des solutions de carbonates et dans le tétrahyàrofurane, la composition suivant l'invention prosure une amélioration sensible des propriétés pharmacocinétiques. En particulier, dans le cas d'une administration par voie orale, sous forme de capsules molles de gélatine ou de gélules contenant la composition de l'invention, on constate une augmentation importante de la vitesse de dissolution et de la concentration maximale obtenue, par comparaison avec une composition usuelle sous forme de gélule.

Les résultats des études cliniques chez l'homme, dans le cas d'une composition en capsules dosée à 38 mg de diacéréine, par comparaison avec une forme galénique classique (gélule) dosée à 50 mg de diacéréine, sont résumés dans le tableau ci-après :

| **Tableau comparatif** | | |
|---|---|---|
| | Gélule | Invention |
| Cₘₐₓ (mg/ml) | 2,07 ± 0,57 | 4,38 ± 1,34 |
| Tₘₐₓ (h) | 4,30 ± 1,65 | 1,30 ± 0,48 |

On voit que la composition suivant la présente invention procure une concentration plasmatique (Cₘₐₓ) nettement augmentée, plus que doublée, en un temps (Tₘₐₓ) bien plus court puisqu'il est ramené de 4,3 heures à 1,3 heure environ. De plus, on constate que les valeurs de la SSC (Surface Sous la Courbe) sont augmentées de plus de 25%. Ces résultats montrent que la biodisponibilité du principe actif utilisé (diacéréine) se trouve nettement améliorée.

Des exemples de compositions et de formes galéniques pour administration orale conformes à la présente invention sont donnés ci-après, pour illustrer l'invention.

### Exemple 1

On prépare une composition contenant :

| | |
|---|---|
| triglycérides à chaîne moyenne | 156 mg |
| huile de soja hydrogénée | 38 mg |
| lécithine de soja | 14,5 mg |
| polysorbate 80 | 43,5 mg |

Cette composition est mélangée soigneusement avec 38 mg de diacéréine dans un mélangeur à disque (400 tours/min.; 30 min.), et encapsulée de manière usuelle dans des capsules à base de gélatine, de glycérine, d'eau purifiée et d'Anidrisorb® additionnée d'oxyde de titane et d'oxyde de fer.

### Exemple 2

Une composition identique à celle de l'Exemple 1 ci-dessus, contenant 38 mg de diacéréine, est encapsulée dans des capsules gélatineuses dont la composition de la paroi comprend les composants indiqués dans l'Exemple 1 ainsi que 15% en poids environ de diméthicone.

### Exemple 3

On prépare une gélule dosée à 30 mg de sel de sodium de rhéine en utilisant une composition contenant :

| | |
|---|---|
| Huile de coco fractionnée | 140 mg |
| huile de soja | 35 mg |
| lécithine de soja | 10 mg |
| Span® (ester de sorbitane) | 30 mg |

Cette composition est mélangée soigneusement avec 30 mg de sel de sodium de rhéine et introduite dans des gélules à paroi de gélatine de type usuel du commerce.

## Revendications

1. Composition pharmaceutique administrable par voie orale, contenant un médicament insoluble ou faiblement soluble dans l'eau et les huiles choisi parmi la diacéréine, la rhéine ou un de leurs sels pharmaceutiquement acceptables, **caractérisée en ce qu'**elle comprend
- une huile liquide support, choisie parmi une huile végétale, animale ou minérale,
- un agent de suspension,
- un agent homogénéiseur,
- un surfactif,
en mélange avec le principe actif du médicament et un ou plusieurs excipients ou supports pharmaceutiquement acceptables.

2. Composition selon la revendication 1, **caractérisée en ce que** l'huile liquide support est un ou plusieurs triglycérides à chaîne moyenne, linéaire ou ramifiée de 8 à 12 atomes de carbone.

3. Composition selon la revendication 1, **caractérisée en ce que** l'agent de suspension est une huile végétale totalement ou partiellement hydrogénée.

4. Composition selon la revendication 3, **caractérisée en ce que** l'huile végétale hydrogénée est l'huile de soja hydrogénée.

5. Composition selon la revendication 1, **caractérisée en ce que** le surfactif est un surfactif non ionique choisi parmi les esters de sorbitane polyalkylés, et les dérivés de polyoxyéthylène sorbitane.

6. Composition selon la revendication 1, **caractérisée en ce que** l'agent homogénéiseur est la lécithine de soja.

7. Composition selon l'une quelconque des revendications précédentes, **caractérisée en ce qu'**elle comprend entre 5 et 10% en poids d'agent homogénéiseur et entre 10 et 15% en poids de surfactif, par rapport au poids total des composants et du principe actif.

8. Composition selon l'une quelconque des revendications précédentes, **caractérisée en ce que** rapport en poids entre l'huile liquide support et l'agent de suspension est compris entre 8:1 et 2:1.

9. Composition selon l'une quelconque des revendications 1 à 8, **caractérisée en ce qu'**elle est présentée sous forme de capsules molles de gélatine.

10. Composition selon l'une quelconque des revendications 1 à 8, **caractérisée en ce qu'**elle est présentée sous forme de gélules.

11. Composition selon l'une quelconque des revendications 9 et 10, **caractérisée en ce que** la paroi de la capsule ou de la gélule contient de la diméthicone.

12. Composition selon l'une quelconque des revendications précédentes, **caractérisée en ce que** le principe actif de médicament est la diacéréine.

## Patentansprüche

1. Oral verabreichbare pharmazeutische Zusammensetzung, die ein in Wasser und in Öl unlösliches oder wenig lösliches Medikament, ausgewählt aus Diacerein, Rhein und pharmazeutisch annehmbaren Salzen davon, enthält, **dadurch gekennzeichnet, dass** sie
- ein flüssiges Trägeröl, ausgewählt aus Pflanzen-, Knochen- und Mineralöl,
- ein Suspendiermittel,
- einen Homogenisierungsmittel,
- ein Tensid
im Gemisch mit dem Wirkstoff des Medikaments und einem oder mehreren Exzipienten oder pharmazeutisch annehmbaren Trägern umfasst.

2. Zusammensetzung nach Anspruch 1, **dadurch gekennzeichnet, dass** das flüssige Trägeröl ein oder mehrere Triglycerid(e) mit verzweigten oder unverzweigten Ketten mit im Schnitt 8 bis 12 Kohlenstoffatomen ist.

3. Zusammensetzung nach Anspruch 1, **dadurch gekennzeichnet, dass** das Suspendiermittel ein zur Gänze oder teilweise hydriertes Pflanzenöl ist.

4. Zusammensetzung nach Anspruch 3, **dadurch gekennzeichnet, dass** das hydrierte Pflanzenöl hydriertes Sojaöl ist.

5. Zusammensetzung nach Anspruch 1, **dadurch gekennzeichnet, dass** das Tensid ein nichtionisches Tensid, ausgewählt aus Estern von polyalkyliertem Sorbitan und Derivaten von Polyoxyethylensorbitan, ist.

6. Zusammensetzung nach Anspruch 1, **dadurch gekennzeichnet, dass** das Homogenisierungsmittel Sojalecithin ist.

7. Zusammensetzung nach einem der vorangegangenen Ansprüche, **dadurch gekennzeichnet, dass** sie zwischen 5 und 10 Gew.-% Homogenisierungsmittel und zwischen 10 und 15 Gew.-% Tensid, bezogen auf das Gesamtgewicht der Bestandteile und des Wirkstoffs, umfasst.

8. Zusammensetzung nach einem der vorangegangenen Ansprüche, **dadurch gekennzeichnet, dass** das Gewichtsverhältnis zwischen dem flüssigen Trägeröl und dem Suspendiermittel zwischen 8:1 und 2:1 liegt.

9. Zusammensetzung nach einem der Ansprüche 1 bis 8, **dadurch gekennzeichnet, dass** sie in Form von Weichgelatinekapseln vorliegt.

10. Zusammensetzung nach einem der Ansprüche 1 bis 8, **dadurch gekennzeichnet, dass** sie in Form von Hartgelatinekapseln vorliegt.

11. Zusammensetzung nach einem der Ansprüche 9 und 10, **dadurch gekennzeichnet, dass** die Hülle der Hartgelatinekapsel oder der Weichgelatinekapsel Dimethicon enthält.

12. Zusammensetzung nach einem der vorangegangenen Ansprüche, **dadurch gekennzeichnet, dass** der Wirkstoff des Medikaments Diacerein ist.

## Claims

1. Pharmaceutical composition which can be administered orally, containing a medicinal product which is insoluble or sparingly soluble in water and oils, chosen from diacerein, rhein or one of their pharmaceutically acceptable salts, **characterized in that** it comprises
- a liquid support oil selected from a plant, animal or mineral oil,
- a suspension agent,
- a homogenizing agent,
- a surfactant,
mixed with the active principle of the medicinal product and one or more pharmaceutically acceptable excipients or supports.

2. Composition according to Claim 1, **characterized in that** the liquid support oil is one or more triglycerides with a linear or branched medium chain of 8 to 12 carbon atoms.

3. Composition according to Claim 1, **characterized in that** the suspension agent is a totally or partially hydrogenated plant oil.

4. Composition according to Claim 3, **characterized in that** the hydrogenated plant oil is hydrogenated soybean oil.

5. Composition according to Claim 1, **characterized in that** the surfactant is a nonionic surfactant chosen from polyalkyl sorbitan esters and polyoxyethylene sorbitan derivatives.

6. Composition according to Claim 1, **characterized in that** the homogenizing agent is soybean lecithin.

7. Composition according to any one of the preceding claims, **characterized in that** it comprises between 5 and 10% by weight of homogenizing agent and between 10 and 15% by weight of surfactant, relative to the total weight of the components and of the active principle.

8. Composition according to any one of the preceding claims, **characterized in that** the weight ratio between the liquid support oil and the suspension agent is between 8:1 and 2:1.

9. Composition according Lo any one of Claims 1 to 8, **characterized in that** it is in the form of soft gelatin capsules.

10. Composition according to any one of Claims 1 to 8, **characterized in that** it is in the form of hard gelatin capsules.

11. Composition according to either of Claims 9 and 10, **characterized in that** the wall of the soft or hard gelatin capsule contains dimethicone.

12. Composition according to any one of the preceding claims, **characterized in that** the active principle of the medicinal product is diacerein.
